Europäisches Patentamt

European Patent Office

Office européen des brevets

(11) Veröffentlichungsnummer: **0 305 577 B1**

(12) **EUROPÄISCHE PATENTSCHRIFT**

(45) Veröffentlichungstag der Patentschrift: 04.12.91

(51) Int. Cl.⁵: **A61F 6/14**

(21) Anmeldenummer: 87112901.1

(22) Anmeldetag: 03.09.87

(54) **Empfängnisverhütende Intrauterinvorrichtung.**

(43) Veröffentlichungstag der Anmeldung:
08.03.89 Patentblatt 89/10

(45) Bekanntmachung des Hinweises auf die
Patenterteilung:
04.12.91 Patentblatt 91/49

(84) Benannte Vertragsstaaten:
CH DE FR GB IT LI

(56) Entgegenhaltungen:
EP-A- 0 117 818
US-A- 4 117 838

(73) Patentinhaber: **NAUCHNO-
PROIZVODSTVENNOE OBIEDINENIE
"MEDINSTRUMENT"
Ulitsa K. Tinchurina, 31
Kazan(SU)**

(72) Erfinder: **Gainutdinova, Raisa Vladimirovna
ulitsa Spartakovskaya, 80, kv. 15.
Kazan(SU)**
Erfinder: **Petrova-Pokrovskaya, Vera Mitrofanovna
ulitsa Frunze, 13a, kv. 2
Kazan(SU)**
Erfinder: **Blokhina, Alevtina Ivanovna
ulitsa Kirova, 15, kv. 40
Kazan(SU)**
Erfinder: **Fridman, Boris Samuilovich
ulitsa Krasnaya Pozitsia, 29b, kv. 83.
Kazan(SU)**
Erfinder: **Gamer, Pinkhos Usherovich
ulitsa R. Zorge, 109, kv. 145
Kazan(SU)**

(74) Vertreter: **Finck, Dieter et al
Patentanwälte v. Füner, Ebbinghaus, Finck
Mariahilfplatz 2 & 3
W-8000 München 90(DE)**

## Beschreibung

Die Erfindung betrifft eine empfängnisverhütende Intrauterinvorrichtung mit einer Leiste mit abgerundeten Enden, mit einem mit der Leiste verbundenen und mit ihr ein T-förmiges Element bildenden Stab, mit einem als elastische Schleife ausgeführten Element zum Halten der Intrauterinvorrichtung innerhalb der Gebärmutterhöhle und mit einer auf dem Stab aufgewickelten Spirale.

Aus der GB-B-14 56 746 ist bereits eine empfängnisverhütende Intrauterinvorrichtung bekannt, die aus einem T-förmigen Element besteht, das von einem Stab und zwei radial abstehenden elastischen Streifen gebildet wird. Am Ende des Elements ist eine elastische Schleife angeordnet. Die gleichgekrümmten Streifen haben jeweils einen ausreichend großen Krümmungsradius. Der Winkel zwischen dem Stab und jedem Streifen liegt unter 90°.

Da der Kontakt der Streifen mit der Schleimhaut des Gebärmuttergrunde nicht ausreichend dicht ist, ist die Kontrazeptionswirkung der Vorrichtung unzureichend. Da die Elastizität der Schleife unzureichend ist und die Schleife sehr groß ist, kann die Schleimhaut im Bereich des inneren Muttermundes geschädigt oder gereizt werden, was zu einer willkürlichen Expulsion der Intrauterinvorrichtung führen kann.

Eine andere empfängnisverhütende Intrauterinvorrichtung (Nova T/Cu 200 Ag der Aktiengesellschaft Khukhtamyaki, Leiras, Finnland) besteht aus einem Stab mit einer daran aufgewickelten Kupferspirale, einer an dem Stab befestigten Leiste mit abgerundeten Enden, die mit dem Stab ein T-förmiges Element bildet, und aus einem als elastische, am Ende des Stabes befestigte Schleife ausgeführten Element zum Halten der ganzen Intrauterinvorrichtung innerhalb der Gebärmutterhöhle.

Aufgrund der Anordnung der elastischen Schleife am Ende des Stabes und aufgrund der Größe und Form der Schleife ist ein ständiges Andrücken der Leiste an die Schleimhaut der Gebärmutter nicht gewährleistet, was die Kontrazeptionswirkung der Vorrichtung nachteilig beeinflußt. Außerdem reizt der Grundbogen der elastischen Schleife die Rezeptoren der Schleimhaut des inneren Muttermunds des Gebärmutterhalses und bewirkt starke Kontraktionen der Muskulatur der Gebärmutter, was zur Expulsion der Vorrichtung führen kann.

Darüber hinaus müssen die bekannten Intrauterinvorrichtungen für die Gebärmutterhöhlen mit verschiedener Größe und Gestalt in unterschiedlichen Baugrößen bereitgestellt werden.

Die der Erfindung zugrundeliegende Vorrichtung besteht darin, die empfängnisverhütende Intrauterinvorrichtung der eingangs genannten Art so auszubilden, daß für Gebärmutterhöhlen unterschiedlicher Größe, Form und Lage jeweils ein und dieselbe Baugröße der Vorrichtung verwendet werden kann.

Diese Aufgabe wird ausgehend von der empfängnisverhütenden Intrauterinvorrichtung der gattungsgemäßen Art siehe z.B. EP-A-0 117 818, die alle Merkmale des Oberbegriffs von Anspruch 1 enthält dadurch gelöst, daß das Element zum Halten der ganzen Intrauterinvorrichtung innerhalb der Gebärmutterhöhle einen Grundbogen und zwei mit dem Grundbogen verbundene elastische Füßchen aufweist, die an der Leiste an der Basis des Stabs befestigt sind, in der von dem Stab und der Leiste gebildeten Ebene liegen und den Stab umfassen.

Zweckmäßigerweise ist der Grundbogen der elastischen Schleife zur Bildung einer Abstützung über der Zervix verstärkt ausgeführt.

Eine derart gebaute empfängnisverhütende Intrauterinvorrichtung ermöglicht es, daß die Leiste daran an die Schleimhaut des Gebärmuttergrundes einer Gebärmutter beliebiger Größe, Form und Stellungslage infolge der elastischen Wirkung des haltenden, aus dem verstärkten Grundbogen und dessen elastischen Füßchen bestehenden Elements angedrückt wird, wodurch die angestrebte Kontrazeptionswirkung gewährleistet wird. Gleichzeitig verhindert das haltende Element aufgrund seines Aufbaus mit dem verstärkten Grundbogen der elastischen Schleife, daß die Intrauterinvorrichtung in den Bereich des inneren Mundes des Gebärmutterhalses eindringt. Sie bleibt in Position über diesem Bereich, ohne Rezeptoren zu verletzten, so daß eine Expulsion vermieden wird.

Anhand einer Zeichnung wird ein Ausführungsbeispiel der Erfindung näher erläutert. Es zeigt:

Fig. 1　die empfängnisverhütende Intrauterinvorrichtung in der Seitenansicht,

Fig. 2　die Vorrichtung eingesetzt in eine Gebärmutterhöhle mit normaler Form und Größe, also mit Idealform,

Fig. 3　in einer Ansicht wie Fig. 2 die Intrauterinvorrichtung eingesetzt in eine Gebärmutterhöhle mit vergrößerter Querabmessung und

Fig. 4　in einer Ansicht wie Fig. 2 die Intrauterinvorrichtung eingesetzt in eine verformte Gebärmutterhöhle mit größerer Längenabmessung.

Die in der Zeichnung gezeigte empfängnisverhütende Intrauterinvorrichtung hat eine Leiste 1 mit abgerundeten Enden und einen mit der Leiste 1 so verbundenen Stab 2, daß die Leiste 1 und der Stab 2 ein T-förmiges Element bilden, wobei der Stab mit der Leiste 1 an seiner Basis verbunden ist. Auf dem Stab 2 ist eine Spirale bzw. Wendel 5 angeordnet. Zum Halten der Intrauterinvorrichtung innerhalb der Gebährmutterhöhle 4 dient ein Element 3, das als elastische Schleife ausgebildet ist. Die elastische Schleife wird von einem verstärkt ausgeführten Grundbogen 6 und zwei mit dem Grundbogen 6 verbundenen elastischen Füßchen 7 gebildet, die an der Leiste 1 neben der Basis des Stabs 2 befestigt sind. Die das Element 3 bildende elastische Schleife liegt in der von dem Stab 2 und der Leiste 1 gebildeten Ebene und umfasst den Stab 2.

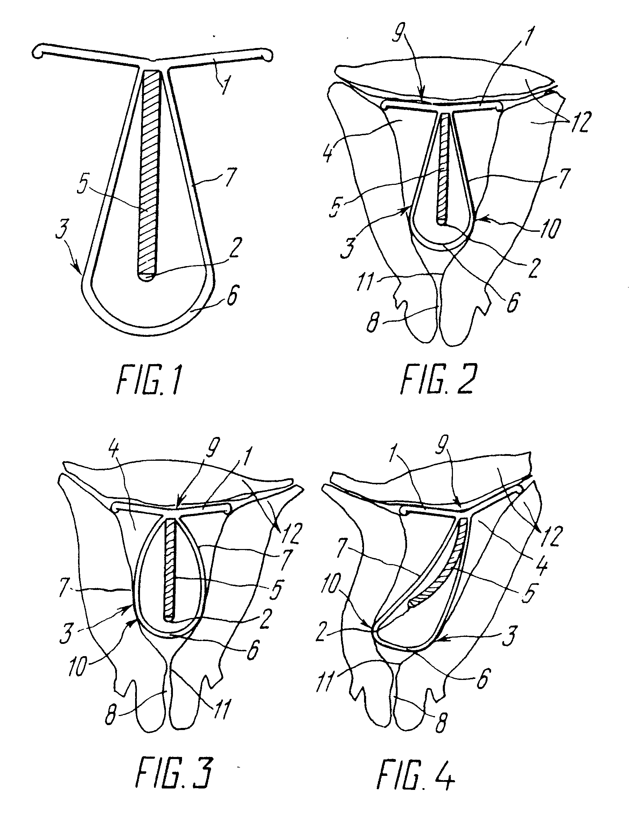

Die Intrauterinvorrichtung wird in die Gebärmutterhöhle 4 über den Mutterhals 8 (Fig. 2) eingeführt und mit der Leiste 1 an die Schleimhaut 9 der Gebärmutter angedrückt. Dabei stößt der verstärkte Grundbogen 6 des Elements 3 gegen die Gebärmutterwand 10 in einem ausreichenden Abstand von dem Bereich der Zervix 11.

Wenn die Intrauterinvorrichtung in eine Gebärmutterhöhle 4 mit größerer Querabmessung, d.h. in eine verkürzte Gebärmutterhöhle 4 eingeführt wird, wie dies in Fig. 3 gezeigt ist, verkürzt sich auch das Element 3 aufgrund seiner Federeigenschaft und hält die Leiste 1 in dichtem Kontakt mit der Schleimhaut 9 der Gebärmutter.

Wenn die Intrauterinvorrichtung in eine Gebärmutterhöhle 4 mit vergrößerter Längsabmessung und verformter Gestalt eingeführt wird, wie dies in Fig. 4 gezeigt ist, wird auch das Element 3 aufgrund seiner Federeigenschaften länger und passt sich der Gestalt der Gebärmutterhöhle 4 so an, daß die Leiste 1 wiederum dicht an die Schleimhaut 9 des Gebärmuttergrundes angedrückt wird.

Der verstärkte Grundbogen 6 des federnden haltenden Elements 3 sorgt für eine zuverlässige Halterung der Intrauterinvorrichtung innerhalb der Gebärmutterhöhle 4 über der Zervix 11 und verhindert eine Expulsion. Durch die federnd elastische Eigenschaft des Elements 3 steht die Leiste 1 der Intrauterinvorrichtung dauern in dichtem Kontakt mit der Schleimhaut 9 des Gebärmuttergrundes und wirkt auf diesen als mechanischer Verschluß ein. Die empfängnisverhütende Intrauterinvorrichtung wird in dieser Ausgestaltung nur in einer einzigen Baugröße benötigt, da sie sich der unterschiedlichen Form, Größe und Position der Gebärmutter anpasst, so daß die gewünschte Kontrazeptionswirkung gewährleistet ist.

## Patentansprüche

1. Empfängnisverhütende Intrauterinvorrichtung mit einer Leiste (1) mit abgerundeten Enden, mit einem mit der Leiste (1) verbundenen und mit ihr ein T-förmiges Element bildenden Stab (2), mit einem als elastische Schleife ausgeführten Element (3) zum Halten der ganzen Intrauterinvorrichtung innerhalb der Gebärmutterhöhle (4) und mit einer auf dem Stab (2) aufgewickelten Spirale (5), dadurch **gekennzeichnet,** daß das Element (3) einen Grundbogen (6) mit zwei mit dem Grundbogen (6) verbundenen elastischen Füßchen (7) ausweist, die an der Leiste (1) im Basisbereich des Stabes (2) befestigt sind, in der von dem Stab (2) und der Leiste (1) gebildeten Ebene liegen und den Stab (2) umfassen.

2. Vorrichtung nach Anspruch 1, dadurch **gekennzeichnet,** daß der Grundbogen (6) der elastischen Schleife zur Bildung einer Abstützung über der Zervix (11) verstärkt ausgeführt ist.

## Claims

1. An intrauterine contraceptive device having a strip (1) with rounded ends, having a bar (2) connected to the strip (1) and forming a T-shaped element with the strip (1), having an element (3) which is constructed as a resilient loop and is for holding the entire intrauterine device within the uterine cavity (4), and having a spiral (5) wound on the bar (2), characterized in that the element (3) has a base curve (6) with two resilient small legs (7) connected to the base curve (6), these small legs being fastened to the strip (1) in the base area of the bar (2), lying in the plane formed by the bar (2) and the strip (1) and surrounding the bar (2).

2. A device in accordance with Claim 1, characterized in that the base curve (6) of the resilient loop is reinforced to form a support over the cervix (11).

## Revendications

1. Dispositif contraceptif intra-utérin avec une barrette (1) à extrémités arrondies, avec une tige (2) reliée à la barrette et constituant un élément en forme de T, avec un élément (3) réalisé sous forme de boucle élastique pour le maintien du dispositif intra-utérin entier à l'intérieur de la cavité de matrice (4) et avec une spirale (5) enroulée sur la tige (2), caractérisé en ce que l'élément (3) comporte un arc de fond (6) et deux branches de pied élastiques (7) reliées à l'arc de fond (6) et qui sont fixées à la barrette (1) dans la région de base de la tige (2), sont situées dans le plan formé par la tige (2) et par la barrette (1) et entourent la tige (2).

2. Dispositif selon la revendication 1, caractérisé en ce que l'arc de fond (6) de la boucle élastique est réalisé de façon renforcée pour la formation d'un appui au-dessus du col de l'utérus (11).

EP 0 305 577 B1

FIG.1

FIG.2

FIG.3

FIG.4

4